Europäisches Patentamt

European Patent Office

Office européen des brevets

(19)

(11) Veröffentlichungsnummer: **0 180 044**
**A1**

(12) # EUROPÄISCHE PATENTANMELDUNG

(21) Anmeldenummer: 85112332.3

(22) Anmeldetag: 28.09.85

(51) Int. Cl.⁴: **C 12 N 1/02, C 12 P 21/00**

(30) Priorität: 10.10.84 DE 3437156

(43) Veröffentlichungstag der Anmeldung: 07.05.86
Patentblatt 86/19

(84) Benannte Vertragsstaaten: AT CH DE FR GB IT LI NL

(71) Anmelder: HOECHST AKTIENGESELLSCHAFT,
Postfach 80 03 20, D-6230 Frankfurt am Main 80 (DE)

(72) Erfinder: Mayer, Manfred, Dr., Königsberger Strasse 8,
D-6272 Niedernhausen/Taunus (DE)
Erfinder: Bennolt, Horst, Klausenerstrasse 90,
D-6620 Völklingen (DE)
Erfinder: Faust, Uwe, Dr., Kelkhelmer Strasse 27,
D-6233 Kelkheim (Taunus) (DE)

(54) Verfahren zur Abtrennung von bakteriellem Bioprotein aus Kulturbrühe.

(57) Bei dem Verfahren zur Abtrennung von bakteriellem Bioprotein aus Kulturbrühe wird der Kulturbrühe ein Lösungsmittel zugegeben. Das Lösungsmittel soll mit Wasser praktisch nicht mischbar und spezifisch leichter als dieses sein. Gleichzeitig mit der Zugabe von Lösungsmittel wird Gas in die Kulturbrühe eingeleitet, um das Bioprotein zu flotieren. Anschließend werden Lösemittel und das flotierte Bioprotein der Flotation zur weiteren Aufarbeitung entnommen.

HOECHST AKTIENGESELLSCHAFT    HOE 84/F 240    DPh.HS/cr

Verfahren zur Abtrennung von bakteriellem Bioprotein aus Kulturbrühe

Vorliegende Erfindung betrifft ein Verfahren zur Abtrennung von bakteriellem Bioprotein aus Kulturbrühe.

Bakterielles Bioprotein wird durch Fermentation gewonnen, bei der z.B. Methanol als Substrat und methanolverwertende Bakterien wie z.B. methylotrophe Stämme des Typs Methylomonas eingesetzt werden.

Die Fermentation liefert üblicherweise eine Kulturbrühe, in der das Bioprotein in einer Konzentration von 1 bis 1,5 Gew.-% und in Teilchengrößen von 0,1 bis 1,5 µm enthalten sind. Weiterhin enthält die Kulturbrühe noch Restgehalte der bei der Fermentation zugesetzten Nährsalze. Die Abtrennung des Bioproteins aus der Kulturbrühe ist ein Teilschritt der Aufbereitung des Bioproteins, das bis auf Restfeuchten an Wasser ◂ 5 Gew.-% getrocknet werden muß.

Bei der Aufarbeitung darf die Biomasse nicht oder höchstens gezielt geschädigt werden. Sie muß außerdem, z.B. für den Einsatz in der Tierernährung, im Organismus biologisch verfügbar und wirksam bleiben. Ferner sollen die Kosten für die Aufarbeitung möglichst niedrig sein und damit dem kommerziellen Einsatz des Produktes nicht im Wege stehen.

Aus verfahrenstechnischer Sicht erweist sich die Aufarbeitung der Kulturbrühe deshalb als besonders schwierig, weil die Bakterienteilchen in sehr feinteiliger Form vorliegen, die Feststoffkonzentration in der Suspension sehr

- 2 -

niedrig und Wasser als Suspensionsflüssigkeit vorgegeben ist, das einerseits nur einen geringen Dichteunterschied zu den Bakterienteilchen hat, andererseits eine hohe Verdampfungsenthalpie aufweist.

Es ist bekannt, solche Kulturbrühen mit 1 bis 1,5 Gew.-% Feststoffanteil in Düsenseparatoren mit hoher Drehzahl und niedrigem spezifischen Durchsatz vorzukonzentrieren. Dabei stellen sich in der eingedickten Suspension Feststoffgehalte von 4 bis 6 Gew.-% ein. Der den Separator verlassende Klarlauf enthält noch ca. 0,1 bis 0,3 g/l nicht abgetrennten Feststoff. Die voreingedickte Suspension wird durch eine Wärmebehandlung bis 80°C und durch eine alkalisch-saure Produktbehandlung zur Flokkulation gebracht, so daß der feinteilige Feststoff unter diesen Bedingungen mit den in einer Vollmantelschneckenzentrifuge erreichbaren Schleuderziffern (ca. 2000 g) sedimentiert und abgetrennt werden kann.

Mit der Vollmantelschneckenzentrifuge, deren Konstruktion auf die sehr scherempfindliche Flocke und deren Beschleunigung unter schonenden Bedingungen Rücksicht nimmt, läßt sich eine Feststoffpaste abtrennen, die max. 30 Gew.-% Feststoff enthält und noch so fließfähig ist, daß sie sich mit einer Verdrängerpumpe fördern und entsprechend weiterverarbeiten läßt. Durch die schonende Behandlung der Mikroflocke wird im Klarlauf der Zentrigue der Feststoffanteil auf Werte von 0,1 Gew.-% und kleiner gehalten. Die Trocknung der Paste erfolgt in einem Zerstäubungstrockner, der aus Gründen der Staubexplosionsfähigkeit des Produktes im Inertgaskreisbetrieb gefahren wird. Der energetische Aufwand für diese Trocknung ist sehr groß; es muß mit einem Energieaufwand pro kg verdampftes Wasser gerechnet werden, der mindetens 2 bis 3 mal so groß ist wie der Wert für die Verdampfungsenthalpie des Wassers. Um den Energieverbrauch auf den ge-

- 3 -

nannten - für die gegebenen Verhältnisse noch relativ niedrigen - Wert zu bringen und einen hohen spezifischen Durchsatz des Trockners zu erzielen, müssen hohe Gaseintrittstemperaturen für den Sprühturm eingestellt werden; dies wiederum kann jedoch leicht zu einer thermischen Produktschädigung führen.

Es bestand demnach die Aufgabe, ein Verfahren zur Abtrennung von Bioprotein aus der Kulturbrühe zu finden, das eine kostengünstige und geringe energieverbrauchende weitere Aufarbeitung des Bioproteins gestattet.

Die Aufgabe wird durch ein Verfahren gelöst, das dadurch gekennzeichnet ist, daß der Kulturbrühe ein mit Wasser praktisch nicht mischbares und spezifisch leichteres Lösemittel zugegeben wird und gleichzeitig mit der Zugabe von Lösemittel das Bioprotein durch Einleiten von Gas in die Kulturbrühe flotiert und zusammen mit dem Lösemittel der Flotation zur weiteren Aufarbeitung entnommen wird.

In einer Ausgestaltung kann das Gas unter Druck in einen Teilstrom von ≥ 5 Vol.-%, vorzugsweise 5 bis 30 Vol.-% Frischwasser bzw. von Bioprotein befreiter Kulturbrühe eingebracht und der mit Gas gesättigte Teilstrom in die Flotation geführt und beim Einleiten entspannt werden. Als Lösemittel eignen sich n-Paraffine, vorzugsweise n-Hexan oder Naphthene, vorzugsweise Cyclopentan oder Cyclohexan. Der Kulturbrühe können 5 bis 30 Vol.-%, vorzugsweise 5 bis 10 Vol.-% Lösemittel zugegeben werden. Die Kulturbrühe kann vor der Flotation zunächst mit Alkali auf einen pH-Wert von 7,5 bis 7,9 gebracht und anschließend mit Säure der pH-Wert soweit abgesenkt werden, daß die elektrophoretische Beweglichkeit der Bioproteinteilchen Null oder nahezu Null ist.

Mit der Zugabe eines geeigneten Lösemittels zur Kulturbrühe kann der Feststoff in die Lösemittelphase überführt werden. Dadurch wird der entscheidende Vorteil erreicht, daß die Abtrennung des Feststoffes aus der Lösemittelphase einfacher vor sich geht und die Trocknung infolge der niedrigeren Verdampfungsenthalpie des Lösemittels und der geringeren Feuchte energetisch weitaus günstiger erfolgen kann. Das Lösemittel soll vorzugsweise wasserunlöslich sein und auch kein Wasser aufnehmen. Eine geringe Wasseraufnahme bzw. geringe Wasserlöslichkeit beeinflussen das erfindungsgemäße Verfahren jedoch nicht.

Sowohl wegen des Siedepunktes als auch wegen des physiologischen Verhaltens sind n-Hexan sowie Cyclohexan und Cyclopentan besonders geeignete Lösemittel.

Die erfindungsgemäße Abtrennung des Bioproteins kann in einer Flotationszelle durchgeführt werden, wobei die Kulturbrühe und das Lösemittel in die Flotationszelle an der gleichen Stelle zugegeben werden. Die Flotation kann durch Gaseinblasen erfolgen. An der Stelle der Einleitung der Kulturbrühe und des Lösemittels, nämlich am Boden der Flotationszelle, ist ebenfalls die Begasungseinrichtung angebracht, mit der gewährleistet wird, daß sich an dieser Stelle feine Gasblasen bilden, die sich an den Feststoff anlagern und ihn an die Flüssigkeitsoberfläche flotieren. Gleichzeitig wird die Flotation des Feststoffes durch das ebenfalls nach oben steigende Lösungsmittel unterstützt. Weit effektiver ist das Flotieren durch die sogenannte Entspannungsflotation am besten in Kombination mit Recyclingstromführung.

In der Flotationszelle bildet sich durch die Lösemittelzugabe eine Zweiphaenschicht aus. Die Oberschicht besteht aus Lösemittel, das überraschenderweise nur den Feststoff enthält und damit das Flotat darstellt. Die Unterphase besteht aus Wasser, das nur noch Spuren an Feststoff und die im Produkt unerwünschten Salze enthält.

Lösemittel und Feststoff werden als Flotat mit Hilfe von Abräumern der Zelle entnommen. Dieser unvorhersehbare Transfer des Feststoffes von der wäßrigen in die organische Phase, ohne daß wesentliche Mengen an Wasser mitgeschleppt werden, hat für die weiteren Aufarbeitungsstufen entscheidende Vorteile. So ist festzustellen, daß der Dekanter bei der Trennung des Lösemittels aus dem abgeräumten Flotat das Sediment mit einem hohen Feststoffanteil von ca. 50 Gew.-% ausbringt und der Klarlauf feststofffrei ist. Die auf den hohen Feststoffanteil konzentrierte Paste läßt sich ohne Schwierigkeiten in einem Kontakttrockner mit besserer Energieausnutzung trocknen. Besonders die niedrigere Anfangsfeuchte der lösemittelhaltigen Paste und die niedrige Verdampfungsenthalpie des Lösemittels bewirken, daß sich der Energieverbrauch pro kg Produkt um eine Größenordnung im Vergleich zur Aufarbeitung der wäßrigen Kulturbrühe ohne Lösemittel verringert. Es kann also ein Kontakttrockner eingesetzt werden, der im Vergleich zum Zerstäubungstrockner weit weniger Platz und keinen Kreisgasbetrieb erfordert. Der aus dem Trockner austretende Brüden wird in einem Kondensator niedergeschlagen.

Die Flotierbarkeit des Feststoffes, d.h. die Agglomerationsfähigkeit der Feststoffteilchen zu Mikroflocken, wird durch die jeweiligen zeitlich sich ändernden Verhältnisse während der Fermentation beeinflußt. So ist es möglich, daß im zeitlichen Verlauf der Fermentation die hergestellte Kulturbrühe mit der beschriebenen Art der Flotation problemlos behandelt werden kann, zu einem zeitlich späteren Zeitpunkt aber keine Flotation mehr zu-

- 6 -

stande kommt. Es wurde jedoch gefunden, daß unabhängig von den zeitlichen, analytisch nicht erfaßbaren Veränderungen im Fermentationsverlauf immer eine Flokkulation und damit eine gute Flotierbarkeit erreicht werden kann, wenn die Kulturbrühe nach folgendem Vorgehen behandelt wird:

Es wird soviel Alkali, z.B. Natronlauge, der Kulturbrühe zugegeben, daß sich ein pH-Wert von 7,5 bis 7,9 einstellt. Danach wird mit Säure, z.B. Schwefelsäure, der pH-Wert soweit erniedrigt, bis die elektrophoretische Beweglichkeit der in der Kulturbrühe suspendierten Teilchen beim Anlegen eines elektrischen Feldes Null oder nahezu Null wird.

Beispiel

Kulturbrühe mit einem Feststoffgehalt von 10 g/l, einem pH-Wert von 6,3 und einer Temperatur von 37°C wurde in einem Rührkessel unter Zugabe von 20 %iger Natronlauge bei einer Verweilzeit von 15 Minuten auf pH 7,6 eingestellt. In einem zweiten Rührkessel wurde mit 20%iger Schwefelsäure der pH-Wert mit einer Verweilzeit von 15 Minuten soweit abgesenkt, bis die elektrophoretische Beweglichkeit U der Bioproteinteilchen bei Null bzw. geringfügig über Null lag (U = 0,1). Der pH-Wert betrug in diesem Fall 3,8. Die elektrophoretische Beweglichkeit wurde mit einer Elektrophorseapparatur des Typs Mark II gemessen. Der Meßwert, nämlich die elektrophoretische Beweglichkeit U wird in Mümeter pro Sekunde dividiert durch Volt pro Zentimeter angegeben. Der U-Wert bei pH 7,6 betrug 3,75; bei pH 3,8 lag der Wert für U bei 0,1.

Die so behandelte Kulturbrühe wurde einer Entspannungsflotation mit Recyclingstrom unterzogen (Fig.). Bei

- 7 -

dieser Art der Flotation wird Flüssigkeit mit Gas unter Druck gesättigt und die gasbeladene Flüssigkeit in der Flotationszelle (2) entspannt. Das freiwerdende Gas bildet dann die für die Flotation notwendigen feinen Gasblasen, die sich an die Mikroflocke anlagern und diese flotieren.

Die vorbehandelte Kulturbrühe, Leitung (3) wurde in die Entspannungszone (8) am Boden einer Flotationszelle (2) eingebracht und gleichzeitig der Recyclingstrom, Leitung (4) über Lochdüsen an der gleichen Stelle eingeleitet. Für den Recyclingstrom (4) wurden 10 Vol.-% des Klarlaufs, Leitung (5) aus der Flotationszelle genommen und mit Luft, Leitung (9) bei 6 bar Überdruck in einem Kessel (1) gesättigt. Mit der über die Lochdüsen entspannten Recyclingflüssigkeit wurde eine Luftmenge freigesetzt, die der Sättigungsdifferenz der Luft in der Flüssigkeit bei den Drücken vor und nach der Entspannung entspricht. Ebenfalls wurde Hexan, Leitung (6) als mit Wasser nicht mischbares Lösemittel am Boden der Flotationszelle (2) zugeführt. Die Lösemittelmenge betrug 10 Vol.-% bezogen auf die zugespeiste Kulturbrühe. An der Oberfläche der Flotationszelle sammelte sich die Flotatschicht, bestehend aus Feststoff und Lösemittel an. Das Flotat, Leitung (7) wurde abgeräumt und anschließend der Feststoff mit einer Zentrifuge (nicht dargestellt) abgetrennt. Der Zentrifugalklarlauf bestand nur aus Hexan und war wasser- und feststofffrei. Das Sediment enthielt 50 Gew.-% Feststoff mit nur sehr geringem Wasseranteil (< 0,1 %). Der wäßrige Klarlauf aus der Flotationszelle hatte nur noch Spuren von Feststoff. (Festgestellt durch Abschleudern der Probe in einer Zentrifuge bei 4500 g).

Das Sediment der Zentrifuge wurde unter Normaldruck und bei einer Heizmitteltemperatur von 100°C in einem Kontakttrockner (nicht dargestellt) auf eine Restfeuchte
< 0,1 Gew.-% getrocknet.

Unter diesen Bedingungen konnte ein Produkt gewonnen werden, das infolge des Produkttransfers vom Wasser ins
Lösungsmittel weit weniger Restgehalte an Salzen hatte
und durch die niedrige und einzige Temperaturbelastung
während der Trocknung schonend und ohne thermische
Schädigung isoliert wurde.

0180044

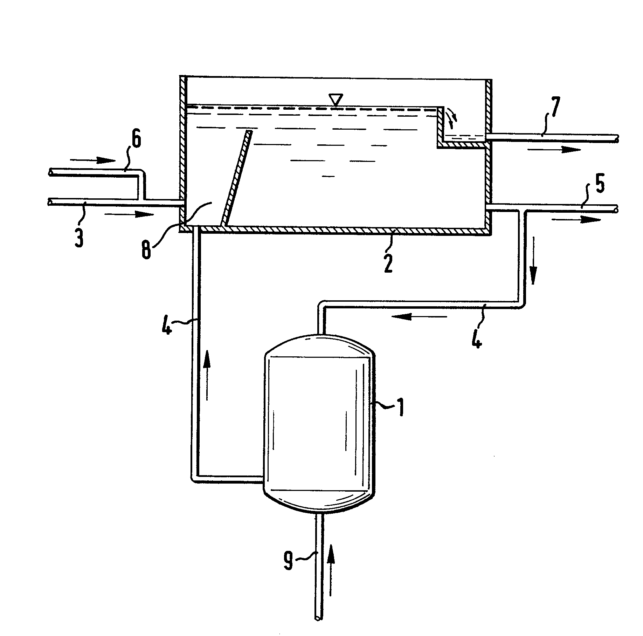

PATENTANSPRÜCHE:

1. Verfahren zur Abtrennung von bakteriellem Bioprotein aus Kulturbrühe, dadurch gekennzeichnet, daß der Kulturbrühe ein mit Wasser praktisch nicht mischbares und spezifisch leichteres Lösemittel zugegeben wird und gleichzeitig mit der Zugabe von Lösemittel das Bioprotein durch Einleiten von Gas in die Kulturbrühe flotiert und zusammen mit dem Lösemittel der Flotation zur weiteren Aufarbeitung entnommen wird.

2. Verfahren nach Anspruch 1, dadurch gekennzeichnet, daß als Lösemittel n-Paraffine, vorzugsweise n-Hexan, oder Naphthene, vorzugsweise Cyclopentan oder Cyclohexan, zugegeben wird.

3. Verfahren nach Anspruch 1, dadurch gekennzeichnet, daß das Gas unter Druck in einen Teilstrom von ⋟ 5 Vol.-% der vom Bioprotein befreiten Kulturbrühe eingebracht und der mit Gas gesättigte Teilstrom als Recyclingstrom in die Flotation zurückgeführt und beim Einleiten entspannt wird.

4. Verfahren nach Anspruch 1, dadurch gekennzeichnet, daß 5 bis 30 Vol.-% Lösemittel in die Kulturbrühe zugegben wird.

5. Verfahren nach Anspruch 1, dadurch gekennzeichnet, daß die Kulturbrühe vor der Flotation zunächst mit Alkali auf einen pH-Wert von 7,5 bis 7,9 gebracht und anschließend mit Säure der pH-Wert soweit abgesenkt wird, daß die elektrophoretische Beweglichkeit der Bioproteinteilchen Null oder nahezu Null ist.

Europäisches
Patentamt

**EUROPÄISCHER RECHERCHENBERICHT**

0180044
Nummer der Anmeldung

EP 85 11 2332

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.4) |
|---|---|---|---|
| X | GB-A-1 177 039 (KYOWA HAKKO KOGYO LTD.) * Seite 2, Zeilen 32-45 und 101-105 * | 1,2 | C 12 N 1/02 C 12 P 21/00 |
| Y | | 3,5 | |
| | --- | | |
| Y | US-A-3 844 893 (DONALD O. HILTZMANN) * Spalte 1, Zeilen 57-64 * | 3 | |
| | --- | | |
| Y | GB-A-1 381 306 (I.C.I. LTD.) * Ansprüche 1 und 2 * | 5 | |
| | --- | | |
| A | FR-A-2 354 967 (SPEPIA ENG.) * Anspruch 2 * | 3 | RECHERCHIERTE SACHGEBIETE (Int. Cl.4) |
| | --- | | |
| A | CH-A- 632 786 (VEBA-CHEMIE) * Seite 3, linke Spalte, Zeilen 1-6 * | 5 | C 12 N |
| | ----- | | |

Der vorliegende Recherchenbericht wurde für alle Patentansprüche erstellt.

| Recherchenort DEN HAAG | Abschlußdatum der Recherche 20-01-1986 | Prüfer MADDOX A.D. |
|---|---|---|

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82